# EUROPÄISCHE PATENTSCHRIFT

(11) **EP 2 905 296 B1**
(45) Veröffentlichungstag und Bekanntmachung des Hinweises auf die Patenterteilung: **23.12.2015**
(21) Anmeldenummer: 14154471.8
(22) Anmeldetag: 10.02.2014
(51) Int. Cl.: C07C 269/02, C07C 271/20, C09D 133/08, C09D 133/06, C08G 18/73, C08G 18/75, C08G 18/32, C08G 18/71, C09D 7/12, C08G 18/80

(54) **Copolymere aus Isocyanatoalkyltrialkoxysilanen und Urethandiolen**
Copolymers made of isocyanatoalkyltrialkoxysilanes and urethane diols
Copolymères constitués d'isocyanatoalkyltrialkoxysilanes et d'uréthanedioles

(43) Veröffentlichungstag der Anmeldung: 12.08.2015
(73) Patentinhaber: Evonik Degussa GmbH, 45128 Essen (DE)
(72) Erfinder: Lomoelder, Rainer, 48153 Münster (DE); Görlitzer, Hans, 44388 Dortmund (DE); Unkelhäußer, Tobias, 45701 Herten (DE); Hallack, Markus, 46514 Schermbeck (DE); Stache, Wiebke, 45657 Recklinghausen (DE)

(56) Entgegenhaltungen:
- EP-A2- 1 721 948
- WO-A1-2009/115079
- WO-A2-2013/189882
- DE-A1- 10 237 270
- GB-A- 2 479 075

## Beschreibung

Die vorliegende Erfindung betrifft Copolymere aus Isocyanatoalkyltrialkoxysilanen und Urethandiolen, Verfahren zur ihrer Herstellung, sie enthaltende Beschichtungsmittel und ihre Verwendung.

An moderne Beschichtungen aller Art, insbesondere von Lackierungen im Automobilbereich, werden hohe Anforderungen gestellt. Zahlreiche Ansätze wurden in der Vergangenheit unternommen, um insbesondere hohe Kratzbeständigkeiten von Lacken, besonders von Decklacken, über Kombinationen aus PUR-Vernetzung und Silan-Vernetzung zu erzielen (WO 2008/074489 A1, WO 2008/110229 A2, WO 2006/042658 A1, WO 2008/110230 A1, EP 1 273 640 A2, DE 10 2004 050 747 A1, US 6,428,898 B1). In der Regel ist die Kratzbeständigkeit abhängig von der Vernetzungsdichte, das heißt von dem Gehalt an Silanmonomeren bzw. -Si(OR)₃-Gruppen. Die relativ hohen Molekulargewichte der im genannten Stand der Technik beschriebenen Vernetzer erfordern jedoch verhältnismäßig geringe Festkörpergehalte der Lackformulierungen, welche zu nachteiligen hohen VOC-Gehalten führt.

Zur Erzielung möglichst hoher Gehalte an -Si(OR)₃-Gruppen eignen sich niedermolekulare Addukte aus linearen oder verzweigten niedermolekularen Diolen sowie Polyether- bzw. Polyester-Polyolen und Isocyanatopropyltrialkoxysilanen. Derartige Addukte sind z. B. in WO 2008/034409 A2, WO 2008/131715 A1 und EP 2 641 925 A1 beschrieben, sowie in WO 2013/189882. Problematisch bei diesen Systemen ist jedoch die unzureichende Flexibilität der resultierenden Beschichtungen. Ein weiteres Problem ist die hohe Kristallisationsneigung und geringe Verträglichkeit von Addukten aus Isocyanatopropyltrialkoxysilanen und niedermolekularen Diolen, so dass bei den vielfach erwünschten Härtungstemperaturen unterhalb von 100 °C mit Verlaufsproblemen und Oberflächenstörungen des resultierenden Lackfilms durch kristallisationsbedingte Unverträglichkeiten der Lackkomponenten zu rechnen ist. Schließlich haben die im Stand der Technik beschriebenen Addukte auch den Nachteil, dass mit ihrem Zusatz hergestellte Beschichtungen nicht über eine hinreichend gute Kratzfestigkeit verfügen.

Es ist somit die Aufgabe der vorliegenden Erfindung, die Nachteile des bekannten Standes der Technik zu vermeiden. Insbesondere ist es Aufgabe der vorliegenden Erfindung, Copolymere bereitzustellen, die als Zusatz in Beschichtungszusammensetzungen zu besonders kratzfesten und gleichzeitig flexiblen Beschichtungen, insbesondere im Automobilbereich, führen.

Diese sich stellende Aufgabe wird vorliegend überraschenderweise gelöst durch die erfindungsgemäßen Copolymere der generischen Formel 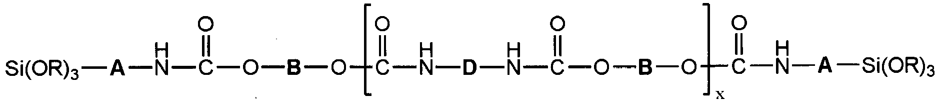 wobei A, B und D jeweils unabhängig voneinander aliphatische (Cyclo)Alkylenreste darstellen, R einen C₁-C₁₀-Alkylrest darstellt und x zwischen 3 und 10 beträgt. Unter (Cyclo)Alkylenresten sind dabei gleichermaßen Cycloalkylenreste und Alkylenreste zu verstehen. Cycloalkylenreste sind im Gegensatz zu den nicht-cyclischen Alkylenresten zweibindige Kohlenwasserstoffreste, die einen nichtaromatischen Kohlenwasserstoffzyklus enthalten oder aus ihm bestehen. Bevorzugt sind die Reste A, B und D Alkylenreste, d.h. zweibindige, nichtzyklische Kohlenwasserstoffreste. Mehrere Reste A am Copolymer können gleiche oder unterschiedliche (Cyclo)Alkylenreste repräsentieren. Bevorzugt repräsentieren mehrere Reste A im Copolymer jedoch denselben (Cyclo)alkylenrest. Mehrere Reste B am Copolymer können gleiche oder unterschiedliche (Cyclo)Alkylenreste repräsentieren. Bevorzugt repräsentieren alle Reste B im Copolymer jedoch denselben (Cyclo)alkylenrest. Mehrere Reste D am Copolymer können gleiche oder unterschiedliche (Cyclo)Alkylenreste repräsentieren. Bevorzugt repräsentieren alle Reste D im Copolymer jedoch denselben (Cyclo)alkylenrest. Auch können die Reste R denselben C₁-C₁₀-Alkylrest darstellen oder für unterschiedliche C₁-C₁₀-Alkylreste stehen. Unter C₁-C₁₀-Alkylresten sind hier und im Folgenden Alkylenreste mit 1 bis 10 Kohlenstoffatomen zu verstehen. Bevorzugt stehen alle Reste R jedoch für denselben C₁-C₁₀-Alkylrest.

Besonders gute Eigenschaften resultieren, wenn jeder Rest A ausgewählt ist aus der Gruppe der linearen C₁-C₁₀-Alkylenreste. Unter C₁-C₁₀-Alkylenresten sind dabei hier und im Folgenden Alkylenreste mit 1 bis 10 Kohlenstoffatomen zu verstehen. Ganz besonders gute Eigenschaften resultieren, wenn jedes A ein Methylen-, Ethylen-, n-Propylen oder n-Butylenrest ist. Ganz besonders bevorzugt ist jedes A ein n-Propylenrest.

Bevorzugt ist weiterhin jeder Rest R ausgewählt aus der Gruppe bestehend aus Methyl, Ethyl, und i-Propyl. Besonders bevorzugt ist jedes R ein Methylrest, da die resultierenden Verbindungen dann besonders reaktiv sind.

Entsprechende Reste R und A können durch entsprechend gewählte Isocyanatoalkyltrialkoxysilane in das erfindungsgemäße Copolymer eingebracht werden. So können entsprechende Reste durch die Verbindungen Isocyanatomethyltrimethoxysilan, Isocyanatomethyltriethoxysilan, Isocyanatomethyltri-iso-propoxysilan, 2-Isocyanatoethyltrimethoxysilan, 2-Isocyanatoethyltriethoxysilan, 2-Isocyanatoethyltri-iso-propoxysilan, 3-Isocyanato-n-propyltrimethoxysilan, 3-Isocyanato-n-propyltriethoxysilan, 3-Isocyanato-n-propyltri-iso-propoxysilan, 4-Isocyanato-n-butyltrimethoxysilan, 4-Isocyanato-n-butyltriethoxysilan und 4-Isocyanato-n-butyltri-iso-propoxysilan eingebracht werden. Ganz bevorzugt ist die Verbindung 3-Isocyanato-n-propyltrimethoxysilan.

Besonders gute Eigenschaften resultieren, wenn jeder Rest B ausgewählt ist aus der Gruppe der linearen, verzweigten oder cyclischen C₁-C₁₂-Alkylenreste. Bevorzugte Reste B sind n-Pentylen, n-Hexylen, n-Dodecylen, 2,4,4-Trimethylhexylen, 2,2,4-Trimethylhexylen 2,2,3-Trimethylpropylen, 1,1,3-Trimethylpropylen, 1,2,3-Trimethylpropylen, 2,2-Methyl-3-isopropyl-propylen, 2-Ethyl- 3-n-propyl-propylen, 2,2-Dimethyl- 3-n-propyl-propylen, 3-Methyl-pentylen, 2-Methyl-pentylen, 2,2-Methyl-propylen, oder cis/trans-1,4-Cyclohexylen. Ganz besonders bevorzugt ist B ein n-Pentylenrest, da die resultierenden Verbindungen zu den besten Ergebnissen führen.

Entsprechende Reste B können über die Wahl entsprechender Diole in das Copolymer eingebracht werden. Bevorzugt werden dafür 1,5-Pentandiol, 1,6-Hexandiol, 1,12-Dodecandiol, 2,2,4-Trimethylhexandiol-1,6, 2,4,4-Trimethylhexandiol-1,6, 2,2-Dimethyl-butandiol-1,3, 2-Methyl-pentandiol-2,4, 3-Methyl-pentandiol-2,4, 2,2,4-Trimethyl-pentandiol-1,3, 2-Ethylhexandiol-1,3, 2,2-Dimethylhexandiol-1,3, 3-Methylpentandiol-1,5, 2-Methyl-pentandiol-1,5, 2,2-Dimethylpropandiol-1,3 (Neopentylglykol) und cis/trans-1,4-Cyclohexandiol (jeweils allein oder als beliebiges Gemisch) eingesetzt.

Besonders gute Eigenschaften resultieren weiterhin, wenn jeder Rest D ein (cyclo)aliphatischer C₆-C₁₅-Alkylenrest ist. Entsprechende Reste können über die Wahl entsprechender Diisocyanate in das Copolymer eingebracht werden. Bevorzugte Diisocyanate, die zu zu besonders vorteilhaften Copolymeren führen, sind die Diisocyanate Isophorondiisocyanat (IPDI), Hexamethylendiisocyanat (HDI), 4,4'-Diisocyanatodicyclohexylmethan (H12MDI), Norbonandiisocyanat (NBDI) sowie 2,2,4-Trimethyl-hexamethylendiisocyanat und 2,4,4-Trimethyl-hexamethylendiisocyanat (als 1:1-Gemisch als TMDI bezeichnet). Entsprechende Reste D haben die nachfolgend dargestellten Strukturformeln:

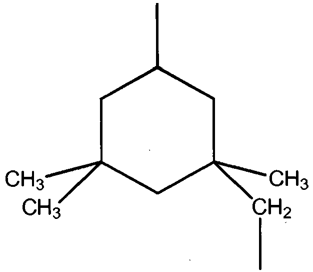

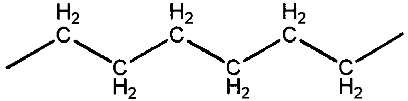

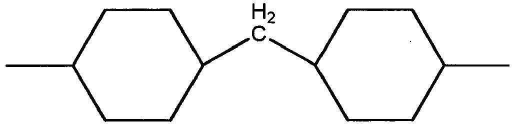

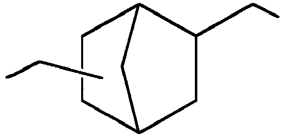

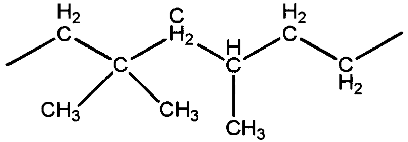

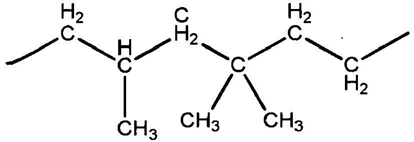

Beste Ergebnisse werden erzielt, wenn der Rest D sich von Isophorondiisocyanat ableitet, d.h. die Strukturformel 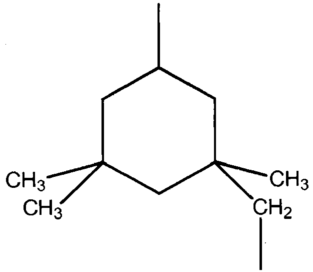 aufweist.

Der Wert für x beträgt einen Wert ausgewählt aus der Werten von 3 bis 10. Bevorzugt beträgt der Wert einen Wert von 4 bis 6. Entsprechende Werte können durch die Reaktionsbedingungen bei der Umsetzung des Diisocyanates mit dem Diol eingestellt werden. Dabei führen die Reduktion der Lösemittelanteils, eine Beschleunigung der Diisocyanat-Zugabe, hohe Temperaturen und die Vorlage des Diisocyanates zu höheren Kettenlängen bzw. zu einer Erhöhung des Wertes x.

Die erfindungsgemäßen Copolymere können über ein Verfahren hergestellt werden, bei dem mindestens ein Diol mit mindestens einem Diisocyanat zu einem Urethanzwischenprodukt umgesetzt wird, welches anschließend mit mindestens einem Isocyanatoalkyltrialkoxysilan umgesetzt wird.

Die Umsetzung des mindestens einen Diols mit dem mindestens einen Diisocyanat erfolgt im Allgemeinen lösemittelfrei oder unter Verwendung von nicht-protischen Lösemitteln. Bevorzugte Lösemittel sind Ethylacetat, Butylacetat, Ethylenglykolmonomethyletheracetat, Ethylenglykolmonoethyletheracetat, 1-Methoxypropyl-2-acetat, 3-Methoxy-n-butylacetat, Aceton, 2-Butanon, 4-Methyl-2-pentanon, Cyclohexanon, Toluol, Xylol, Chlorbenzol, Testbenzin, höher substituierte Aromaten (wie sie beispielsweise unter den Bezeichnungen Solventnaphtha, Solvesso®, Isopar® und Nappar® der Deutsche EXXON CHEMICAL GmbH bzw. als Shellsol® der Deutsche Shell Chemie GmbH im Handel sind), Kohlensäureester (insbesondere Dimethylcarbonat, Diethylcarbonat, 1,2-Ethylencarbonat und 1,2-Propylencarbonat), Lactone (insbesondere Propiolacton, Butyrolacton, Caprolacton und Methylcaprolacton), Propylenglykoldiacetat, Diethylenglykoldimethylether, Dipropylenglykoldimethylether, Diethylenglykolethyletheracetat, Diethylenglykolbutyletheracetat, N-Methylpyrrolidon und N-Methylcaprolactam. Die vorgenannten Lösemittel können alleine in beliebigen Gemischen eingesetzt werden. Besonders bevorzugt wird Butylacetat eingesetzt. Die Reaktion wird bevorzugt unter Ausschluss von Wasser durchgeführt.

Die Umsetzung kann diskontinuierlich oder kontinuierlich erfolgen. Die Reaktion kann bei Raumtemperatur, das heißt bei Temperaturen im Bereich von 20 - 25 °C, geführt werden, bevorzugt werden jedoch höhere Temperaturen im Bereich von 30 - 150 °C, insbesondere im Bereich von 50 - 150 °C verwendet. Zur Beschleunigung der Reaktion können vorteilhaft in der Urethanchemie bekannte Katalysatoren eingesetzt werden. Bevorzugt werden als Katalysatoren mindestens ein tertiäres oder aromatisches Amin (insbesondere Triethylamin, Pyridin, Methylpyridin, Benzyldimethylamin, N,N-Endoethylenpiperazin, N-Methylpiperidin, Pentamethyldiethylentriamin, N,N-Dimethylaminocyclohexan, N,N'-Dimethylpiperazin) und/oder mindestens ein Metallsalz (insbesondere Eisen(II)-chlorid, Aluminiumtri-(ethylacetoacetat), Zinkchlorid, Zink(II)-n-octanoat, Zink(II)-2-ethyl-1-hexanoat, Zink(II)-2-ethylcaproat, Zink(II)-stearat, Zink(II)-naphthenat, Zink(II)-acetylacetonat, Zinn(II)-n-octanoat, Zinn(II)-2-ethyl-1-hexanoat, Zinn(II)-ethylcaproat, Zinn(II)-laurat, Zinn(II)-palmitat, Dibutylzinn(IV)-oxid, Dibutylzinn(IV)-dichlorid, Dibutylzinn(IV)-diacetat, Dibutylzinn(IV)-dimaleat, Dibutylzinn(IV)-dilaurat, Dioctylzinn(IV)-diacetat, Molybdänglykolat) eingesetzt. Werden Katalysatoren eingesetzt, werden diese bevorzugt in einer Konzentration im Bereich von 0,001 bis 2 Gew.-%, vorzugsweise im Bereich von 0,005 bis 0,5 Gew.-%, bezogen auf das Gesamtgewicht der Reaktionspartner, eingesetzt.

Bevorzugte Diole sind 1,5-Pentandiol, 1,6-Hexandiol, 1,12-Dodecandiol, 2,2,4-Trimethylhexandiol-1,6 und 2,4,4-Trimethylhexandiol-1,6 allein oder als beliebige Gemische dieser Isomeren, 2,2-Dimethyl-butandiol-1,3, 2-Methyl-pentandiol-2,4, 3-Methyl-pentandiol-2,4, 2,2,4-Trimethyl-pentandiol-1,3, 2-Ethylhexandiol-1,3, 2,2-Dimethylhexandiol-1,3, 3-Methylpentandiol-1,5, 2-Methyl-pentandiol-1,5, 2,2-Dimethylpropandiol-1,3 (Neopentylglykol) und cis/trans-1,4-Cyclohexandiol. Vorzugsweise weisen die Diole ein Molekulargewicht von 76 bis 314 g/mol, weiter bevorzugt von 90 bis 206 g/mol auf.

Bevorzugte Diisocyanate sind Isophorondiisocyanat (IPDI), Hexamethylendiisocyanat (HDI), 4,4'-Diisocyanatodicyclohexylmethan (H12MDI), Norbonandiisocyanat (NBDI), sowie 2,2,4-Trimethyl-hexamethylen-diisocyanat und 2,4,4-Trimethyl-hexamethylendiisocyanat (als 1:1-Gemisch als TMDI bezeichnet).

Die Umsetzung des mindestens einen Diols mit dem mindestens einen Diisocyanat zur Bildung der Urethanzwischenprodukte erfolgt dabei bevorzugt derart, dass das Verhältnis der OH-Gruppen der Diole zu der Summe der NCO- Gruppen der Diisocyanate 1:1 bis 5:1, weiter bevorzugt 1,5:1 bis 3:1, beträgt. Ganz besonders bevorzugt ist das Umsetzungsverhältnis 2:1. Somit erfolgt insbesondere bevorzugt eine vollständige Umsetzung aller NCO-Gruppen der Diisocyanate mit OH-Gruppen der Diole.

Das erhaltene Urethanzwischenprodukt wird anschließend mit mindestens einem Isocyanatoalkyltrialkoxysilan umgesetzt. Dabei kann das Urethanzwischenprodukt mit oder ohne zuvor erfolgende Isolierung eingesetzt werden. Bevorzugt wird das Urethanzwischenprodukt ohne zuvor erfolgende Isolierung oder Aufreinigung eingesetzt. Bevorzugte Isocyanatoalkyltrialkoxysilane sind Isocyanatomethyltrimethoxysilan, Isocyanatomethyltriethoxysilan, Isocyanatomethyltri-iso-propoxysilan, 2-Isocyanatoethyltrimethoxysilan, 2-Isocyanatoethyltriethoxysilan, 2-Isocyanatoethyltri-iso-propoxysilan, 3-Isocyanato-n-propyltrimethoxysilan, 3-Isocyanato-n-propyltriethoxysilan, 3-Isocyanato-n-propyltri-iso-propoxysilan, 4-Isocyanato-n-butyltrimethoxysilan, 4-Isocyanato-n-butyltriethoxysilan und 4-Isocyanato-n-butyltri-iso-propoxysilan. Ganz besonders bevorzugt ist 3-Isocyanato-n-propyltrimethoxysilan.

Die Umsetzung der Urethanzwischenprodukte mit den Isocyanatoalkyltrialkoxysilanen zur Bildung der erfindungsgemäßen Copolymere erfolgt weiterhin bevorzugt derart, dass das Verhältnis von OH-Gruppen der Urethanzwischenprodukte zu NCO-Gruppen der Isocyanatoalkyltrialkoxysilane bevorzugt 0,8:1 bis 1,2:1, weiter bevorzugt 0,9:1 bis 1,1:1, beträgt. Ganz besonders bevorzugt ist die stöchiometrische Umsetzung. Somit erfolgt insbesondere ganz besonders bevorzugt eine vollständige Umsetzung aller OH-Gruppen der Diole mit den NCO-Gruppen der Verbindungen der Isocyanatoalkyltrialkoxysilane.

Bei der genannten Umsetzung reagieren die NCO-Gruppen der Isocyanatoalkyltrialkoxysilane mit den OH-Gruppen der Diole unter Ausbildung von -NH-CO-O-Gruppen, die diese Verbindungen miteinander verknüpfen.

Die Umsetzung des mindestens einen Isocyanatoalkyltrialkoxysilans mit dem Urethanzwischenprodukt erfolgt im Allgemeinen unter den gleichen Bedingungen wie oben für die Umsetzung des mindestens einen Diols mit dem mindestens einen Diisocyanat beschrieben.

Die erfindungsgemäßen Copolymere sind insbesondere bei Temperaturen oberhalb von 20 °C flüssig. In Abhängigkeit der gewählten Stöchiometrie beider Reaktionspartner kann das Reaktionsprodukt noch freie Hydroxyl- oder Isocyanatgruppen enthalten. Bevorzugt sind die erfindungsgemäßen Addukte jedoch im Wesentlichen frei von Hydroxylgruppen. Die erfindungsgemäßen Addukte sind bevorzugt in lösemittelfreier Form mittel- bis hochviskos und bei 20 °C flüssig. Zwecks besserer Handhabbarkeit können die Produkte jedoch mit nicht protischen Lösemitteln, versetzt sein. Die Festkörpergehalte derartiger Zubereitungen betragen 50 -100%, vorzugsweise > 80 Gew.-% und weisen dabei eine bevorzugt eine Viskosität von 2 - 80 Pas (DIN EN/ISO 3219 23 °C) auf.

Die erfindungsgemäßen Copolymere werden in vorteilhafter Weise als vernetzende Komponente für kratzbeständige und flexible Klarlacke verwendet. Dabei werden sie zur Optimierung der Lackmechanik mit polymeren Bindemitteln, die auch vernetzbare funktionelle Gruppen tragen können, abgemischt. Die Reaktivität der erfindungsgemäßen Copolymere kann jedoch für eine Aushärtungsgeschwindigkeit bei Umgebungstemperatur nicht ausreichend sein. Zur Steigerung der Vernetzungsgeschwindigkeit können daher vorzugsweise Katalysatoren zugesetzt und/oder die Aushärtung bei Temperaturen oberhalb Umgebungstemperatur durchgeführt werden.

Hierzu eignen sich Metall- oder Übergangsmetallchelate, -Salze oder -Partikel, beispielsweise auf Basis von Titan-, Aluminium-, Zinn- oder Zirkoniumkomplexen, Sulfonsäuren, Phosphorsäure oder phosphorigen Säuren und deren Derivaten, Carbonsäuren mit Schmelzpunkten oberhalb von 60 °C, quarternäre Ammoniumcarboxylate oder auch Kombinationen der genannten Verbindungen.

Die bei der erfindungsgemäßen Verwendung einzusetzenden Lacke können lösemittelfrei oder lösemittelhaltig sein. Insbesondere bevorzugt sind die einzusetzenden Lacke nicht-wässrig. Nicht wässrig im Sinne der vorliegenden Erfindung meint einen Gehalt an Wasser im Lack von nicht mehr als 1,0 Gew.-%, vorzugsweise nicht mehr als 0,5 Gew.-%, bezogen auf den Lack. Insbesondere im Falle zweikomponentiger Formulierungen kann die vorab genannte geringe Menge Wasser zur Beschleunigung der Aushärtung eingesetzt werden. Insbesondere bevorzugt ist das eingesetzte Lacksystem frei von Wasser (maximal 500 ppm Wasser).

Die mittels der erfindungsgemäßen Addukte erhältlichen Lacke können insbesondere zur Beschichtung von Holz, Papier, Kunststoff, Glas, Textilien oder Metall eingesetzt werden. Auf diese Weise werden hoch kratzfeste und bereits bei Temperaturen unterhalb von 100 °C vernetzende Lacke erhalten.

Somit ist ein weiterer Gegenstand der Erfindung die Verwendung der erfindungsgemäßen Copolymere als Beschichtungsmittel oder als Bestandteil von Beschichtungsmitteln, insbesondere zur Herstellung kratzbeständiger und flexibler Klarlacke. Insbesondere bevorzugt sind die bei Temperaturen oberhalb von 0 °C flüssigen Copolymere.

Die auf Basis der vorab genannten Beschichtungsmittel erhaltenen Beschichtungen sind gekennzeichnet durch eine hohe Beständigkeit gegenüber mechanischer Beanspruchung, insbesondere weisen sie eine hohe Kratzbeständigkeit auf. Überraschend dabei ist, dass die erhaltenen Beschichtungen gleichzeitig eine besonders hohe Flexibilität und weiterhin einen hohen Glanz aufweisen.

Weiterer Gegenstand der vorliegenden Erfindung sind Beschichtungsmittel, welche vorzugsweise bei Temperaturen von 20 bis 150 °C härtbar sind, enthaltend
A) mindestens ein erfindungsgemäßes Copolymer,
B) eine oder mehrere Bindemittelkomponenten,
C) optional bis zu 4 Gew.-% mindestens eines Katalysators,
D) optional Hilfs- und Zusatzstoffe,
E) optional organische Lösemittel.

Der Anteil der erfindungsgemäßen Copolymere als Komponente A) in dem erfindungsgemäßen Beschichtungsmittel beträgt bevorzugt 30-90 Gew.-%, besonders bevorzugt 20 bis 80 Gew.-%, bezogen auf das Beschichtungsmittel.

Weiterhin kann das erfindungsgemäße Beschichtungsmittel optional eine oder mehrere Bindemittel-Komponenten B enthalten. Grundsätzlich eignen sich als Bindemittel-Komponenten alle dem Fachmann bekannten Arten von Bindemitteln, beispielsweise auch thermoplastische, das heißt nicht vernetzbare Bindemittel, die üblicherweise ein mittleres Molekulargewicht > 10000 g/mol aufweisen. Bevorzugt werden jedoch Bindemittel, die über reaktive funktionelle Gruppen mit aciden Wasserstoffatomen verfügen, eingesetzt. Geeignete Bindemittel der genannten Art weisen beispielsweise mindestens eine, bevorzugt jedoch zwei oder mehr Hydroxylgruppen auf. Weitere geeignete funktionelle Gruppen des Bindemittels sind beispielsweise Trialkoxysilan-Funktionalitäten.

Als Bindemittel mit funktionellen Gruppen werden bevorzugt Hydroxylgruppen-haltige Polymere, insbesondere Hydroxylgruppen-haltige Polyester, Polyether, Poly(meth)acrylate, Polycarbonate und Polyurethane mit einer OH-Zahl von 20 bis 500 mg KOH/g und einer mittleren Molmasse von 250 bis 6000 g/Mol eingesetzt. Besonders bevorzugt werden im Rahmen der vorliegenden Erfindung Hydroxylgruppen-haltige Polyester oder Poly(meth)acrylate mit einer OH-Zahl von 20 bis 150 mg KOH/g und einem mittleren Molekulargewicht von 500 bis 6000 g/mol als Bindemittelkomponenten eingesetzt. Unter Poly(meth)acrylaten sind dabei sowohl Polyacrylate als auch Polymethacrylate zu verstehen. Die Hydroxylzahl (OHZ) wird bestimmt nach DIN 53240-2. Bei diesem Verfahren wird die Probe mit Essigsäureanhydrid in Gegenwart von 4-Dimethylaminopyridin als Katalysator umgesetzt, wobei die Hydroxylgruppen acetyliert werden. Dabei entsteht pro Hydroxylgruppe ein Molekül Essigsäure, während die anschließende Hydrolyse des überschüssigen Essigsäureanhydrids zwei Moleküle Essigsäure liefert. Der Verbrauch an Essigsäure wird titrimetrisch aus der Differenz zwischen Hauptwert und einem parallel durchzuführenden Blindwert ermittelt. Das Molekulargewicht wird bestimmt mittels Gelpermeationschromatographie (GPC). Die Charakterisierung der Proben erfolgte in Tetrahydrofuran als Eluent nach DIN 55672-1.

Als hydroxylgruppenhaltige (Meth)acryl-Copolymerisate können Harze mit einer Monomerzusammensetzung, wie sie z. B. in WO 93/15849 (S. 8, Zeile 25 bis S. 10, Zeile 5), oder auch in DE 195 29124 beschrieben sind, verwendet werden. Dabei sollte die durch anteilige Verwendung von (Meth)acrylsäure als Monomer einzustellende Säurezahl des (Meth)acryl-Copolymerisates 0 - 30, vorzugsweise 3 - 15 mg KOH/g betragen. Das zahlenmittlere Molgewicht (ermittelt durch Gelpermeationschromatographie gegen einen Polystyrolstandard) des (Meth)acryl-Copolymerisates beträgt vorzugsweise 2000 - 20000 g/mol, die Glasübergangstemperatur beträgt vorzugsweise -40 °C bis +60 °C. Der durch anteilige Verwendung von Hydroxyalkyl(meth)acrylaten einzustellende Hydroxylgehalt der erfindungsgemäß zu verwendenden (Meth)acryl-Copolymerisate beträgt vorzugsweise 70 - 250 mg KOH/g, besonders bevorzugt 90 - 190 mg KOH/g.

Erfindungsgemäß geeignete Polyesterpolyole sind Harze mit einer Monomerzusammensetzung aus Di- und Polycarbonsäuren und Di- und Polyolen, wie sie z. B. in Stoye/Freitag, Lackharze, C. Hanser Verlag, 1996, S. 49 oder auch in WO 93/15849 beschrieben sind. Als Polyesterpolyole können auch Polyadditionsprodukte von Caprolacton an niedermolekulare Di- und Triole, wie sie z. B. unter der Bezeichnung CAPA (Perstorp) erhältlich sind, eingesetzt werden. Das rechnerisch bestimmte zahlenmittlere Molgewicht beträgt vorzugsweise 500 - 5000 g/mol, besonders bevorzugt 800 - 3000 g/mol, die mittlere Funktionalität beträgt vorzugsweise 2,0 - 4,0, bevorzugt 2,0 - 3,5.

Als erfindungsgemäß zu verwendende urethan- und estergruppenhaltige Polyole kommen prinzipiell auch solche zum Einsatz, wie sie in EP 140 186 beschrieben sind. Bevorzugt werden urethan- und estergruppenhaltige Polyole, zu deren Herstellung HDI, IPDI, Trimethylhexamethylendüsocyanat (TMDI) oder H₁₂-MDI verwendet werden, eingesetzt. Das zahlenmittlere Molgewicht beträgt vorzugsweise 500 - 2000 g/mol, die mittlere Funktionalität liegt insbesondere im Bereich von 2,0 - 3,5

Auch Trialkoxysilan-funktionelle Bindemittel eignen sich zur Verwendung als Komponente B. Derartige Harze können gewonnen werden durch Copolymerisation von Acrylat- oder Methacrylat-Monomeren mit acryl- oder methacryl-funktionellen Alkyl-Trialkoxysilan-Derivaten (z. B. Dynasylan^{®} MEMO der Evonik Industries AG), wie sie zum Beispiel in WO 92/11328 beschrieben sind. Ein alternativer Syntheseweg besteht in der Derivatisierung von hydroxylgruppenhaltigen Polyethern, Polyestern, Polycarbonat-diolen oder Polyacrylaten mit Isocyanatopropyltrialkoxysilan, wie er beispielsweise in WO 2008/131715 in den Beispielen 3 und 4 beschrieben ist.

Selbstverständlich können auch Mischungen der vorab beschriebenen Bindemittel eingesetzt werden. Bevorzugte Bindemittel sind Hydroxylgruppen-haltige Polyester und Polyacrylate, allein oder in Mischungen.

Der Anteil von B) in dem erfindungsgemäßen Beschichtungsmittel beträgt bevorzugt 10-80 Gew.-%, bezogen auf das Beschichtungsmittel, insbesondere 20 bis 80 Gew.-%.

Das Massenverhältnis der Komponente A) zu der Komponente B) beträgt in dem erfindungsgemäßen Beschichtungsmittel vorzugsweise 3:7 bis 7:3.

Zur Erzielung einer ausreichenden Aushärtungsgeschwindigkeit bei Aushärtungstemperaturen von weniger als 100 °C werden vorzugsweise Katalysatoren C) eingesetzt. Geeignete Katalysatoren sind insbesondere Lewis-Säuren, Metall- oder Übergangsmetallchelate, -Salze oder -Partikel, beispielsweise auf Basis von Titan-, Aluminium-, Zinn- oder Zirkoniumkomplexen, Sulfonsäuren in freier aber auch neutralisierter oder adduktierter Form, wie sie beispielsweise in DE 2356768 beschrieben sind, Phosphorsäure oder phosphorige Säuren und deren Derivate (WO 2008/074491, Seite 18, Zeilen 1 - 17), hoch siedende Säuren, quarternäre Ammoniumcarboxylate oder auch Kombinationen der genannten Verbindungen. Bevorzugt werden Übergangsmetallchelate oder -salze, hoch siedende Säuren, quarternäre Ammoniumcarboxylate oder Kombinationen der genannten Verbindungen eingesetzt.

Besonders bevorzugt ist die Komponente C) mindestens ein Katalysator ausgewählt aus der Gruppe C1) der organischen Carbonsäuren mit einem Schmelzpunkt oberhalb von 60 °C und/oder der Gruppe C2) der Tetraalkylammoniumcarboxylate.

Geeignete organische Carbonsäuren mit einem Schmelzpunkt oberhalb von 60 °C (bei Normaldruck) sind bei Raumtemperatur nicht flüchtige Verbindungen. Beispiele vorteilhaft einzusetzender Carbonsäuren sind Salicylsäure, Benzoesäure, Citronensäure, Isophthalsäure, Phthalsäure, Terephthalsäure und/oder Trimellithsäure. Bevorzugt werden im Rahmen der vorliegenden Erfindung Salicylsäure und Benzoesäure verwendet.

Als Katalysator C2) wird ein Tetraalkylammoniumcarboxylat eingesetzt. Beispiele hierfür sind Tetramethylammoniumformiat, Tetramethylammoniumacetat, Tetramethylammoniumpropionat, Tetramethylammoniumbutyrat, Tetramethylammoniumbenzoat, Tetraethylammoniumformiat, Tetraethylammoniumacetat, Tetraethylammoniumpropionat, Tetraethylammoniumbutyrat, Tetraethylammoniumbenzoat, Tetrapropylammoniumformiat, Tetrapropylammoniumacetat, Tetrapropylammoniumpropionat, Tetrapropylammoniumbutyrat, Tetrapropylammoniumbenzoat, Tetrabutylammoniumformiat, Tetrabutylammoniumacetat, Tetrabutylammoniumpropionat, Tetrabutylammoniumbutyrat und/oder Tetrabutylammoniumbenzoat. Die genannten Tetraalkylammoniumcarboxylate können allein oder in Mischungen zugesetzt werden. Bevorzugt werden Tetraethylammoniumbenzoat und/oder Tetrabutylammoniumbenzoat eingesetzt.

Die Katalysatorkomponente C) in den erfindungsgemäßen Beschichtungsmitteln kann allein aus den oben genannten Alternativen C1) oder C2) bestehen, es können aber auch beliebige Mischungen der Katalysatoren C1) und C2) eingesetzt werden. Derartige Mischungen aus C1) und C2) weisen insbesondere ein Verhältnis von 9 : 1 bis 1 : 9 (m/m) auf. Vorzugsweise beträgt der Anteil der Komponente C) bis zu 4 Gew.-%, bezogen auf das Beschichtungsmittel, vorzugsweise 0,1 bis 4 Gew.-%.

Das erfindungsgemäße Beschichtungsmittel kann zusätzlich in der Lacktechnologie bekannte Hilfsstoffe und/oder Zusatzstoffe D) wie Stabilisatoren, Lichtschutzmittel, Katalysatoren, Füllstoffe, Pigmente, Verlaufsmittel oder Rheologiehilfsmittel, wie z. B. sog. "sag control agents", Mikrogele oder pyrogenes Siliciumdioxid, in typischen Konzentrationen enthalten. Falls erforderlich, können in der Komponente D) der erfindungsgemäßen Beschichtungsmittel auch in der Lacktechnologie übliche anorganische oder organische Farb- und/oder Effektpigmente eingearbeitet werden.

Die Komponente D) ist im Falle von pigmentfreien Beschichtungsmitteln, d. h. Klarlacken, vorzugsweise in Mengen von 0,5 bis zu 8 Gew.-%, insbesondere 1 bis 6 %, bezogen auf das Beschichtungsmittel, in dem erfindungsgemäßen Beschichtungsmittel enthalten. Im Falle von pigment- und/oder füllstoffhaltigen Beschichtungsmitteln kann der Gehalt von Komponente D) 5 bis 80 Gew.-%, insbesondere 10 bis 70 Gew.-% betragen, bezogen das Beschichtungsmittel.

Weiterhin kann das erfindungsgemäße Beschichtungsmittel organische Lösemittel als Komponente E) enthalten. Geeignete Lösemittel sind z. B. Ketone, Ester, Alkohole oder Aromaten.

Die Komponente E) ist vorzugsweise in Mengen von 20 bis zu 60 Gew.-%, insbesondere 20 % bis 50 %, bezogen auf das Beschichtungsmittel, in dem erfindungsgemäßen Beschichtungsmittel enthalten. Die Menge der Komponente E) orientiert sich an der einzustellenden Applikationsviskosität des Beschichtungsmittels.

Die Summe aller Anteile der Komponenten A) bis E) ergibt 100 Gew.-%. Bevorzugt bestehen die erfindungsgemäßen Beschichtungsmittel aus den genannten Komponenten A) bis E).

Weiterer Gegenstand der vorliegenden Erfindung sind vorzugsweise bei Temperaturen von 0° bis 40 °C härtbare Beschichtungsmittel enthaltend
I) mindestens ein erfindungsgemäßes Copolymer,
II) optional mindestens ein Addukt aus mindestens einem Isocyanatosilan und mindestens einer Hydroxy-funktionellen Verbindung,
III) mindestens eine Zinn-haltige Verbindung und
IV) mindestens ein Aminosilan.

Das erfindungsgemäße Beschichtungsmittel kann somit mindestens ein erfindungsgemäßes Copolymer, mindestens eine Zinn-haltige Verbindung und mindestens ein Aminosilan enthalten. Es kann weiter bevorzugt mindestens ein erfindungsgemäßes Copolymer, mindestens ein Addukt aus mindestens einem Isocyanatosilan und mindestens einer Hydroxy-funktionellen Verbindung, mindestens eine Zinn-haltige Verbindung und mindestens ein Aminosilan enthalten. Bevorzugt besteht das erfindungsgemäße Beschichtungsmittel aus mindestens einem erfindungsgemäßen Copolymer, mindestens einer Zinn-haltigen Verbindung und mindestens einem Aminosilan oder aus mindestens einem erfindungsgemäßen Copolymer, mindestens einem Addukt aus mindestens einem Isocyanatosilan und mindestens einer Hydroxy-funktionellen Verbindung, mindestens einer Zinn-haltigen Verbindung und mindestens einem Aminosilan. Besonders bevorzugt besteht das erfindungsgemäße Beschichtungsmittel aus einem erfindungsgemäßen Copolymer, einer Zinn-haltigen Verbindung und einem Aminosilan oder aus einem erfindungsgemäßen Copolymer, einem Addukt aus mindestens einem Isocyanatosilan und mindestens einer Hydroxy-funktionellen Verbindung, einer Zinn-haltigen Verbindung und einem Aminosilan.

Es hat sich überraschenderweise gezeigt, dass Beschichtungsmittel aus diesen Komponenten bereits bei 0°C zu stabilen Beschichtungen führen. Dabei handelt es sich bei diesen erfindungsgemäßen Beschichtungsmitteln um einkomponentige Systeme, die einfach zu applizieren sind. Sie haben weiterhin den Vorteil, aufgrund der niedermolekularen Anteile der Beschichtungsmittel im Hinblick auf eine spätere Anwendung ohne zusätzliche organische Lösungsmittel formulierbar und verarbeitbar zu sein. Somit ist es insbesondere möglich, einen VOC-Gehalt von unter 100 g/l zu realisieren. Die Menge an Komponenten I) und II) in dem erfindungsgemäßen Beschichtungsmittel beträgt bevorzugt 10 bis 90 Gew.-%, insbesondere bevorzugt 10 bis 80 Gew.-%, jeweils bezogen auf die Gesamtmasse des Beschichtungsmittels.

Bei der Komponente III) der erfindungsgemäßen Beschichtungsmittel handelt es sich um eine Zinn-haltige Verbindung, bevorzugt um eine Organozinnverbindung. Besonders bevorzugt handelt es sich um mindestens eine organische Zinnverbindung der Formel R¹₄₋ₐSnXₐ, wobei a 1, 2 oder 3 ist, R¹ unabhängig ausgewählt wird aus der Gruppe bestehend aus linearen oder verzweigten, gegebenenfalls substituierten, C₁-C₃₀-Alkylgruppen-, C₅-C₁₄-Cycloalkylgruppen oder C₆-C₁₄-Arylgruppen, Triorganylsilyl- sowie C₁-C₃₀-Diorganylalkoxysilyl-Gruppen, und X ausgewählt wird aus der Gruppe bestehend aus Halogen, -OR², -OC(O)R³, -OH, -SR⁴, -NR⁵₂, - NHR⁶, -OSiR⁷₃, -OSi(OR⁸)₃, worin die Substituenten R² bis R⁸ jeweils unabhängig voneinander ausgewählt werden aus gegebenenfalls substituierten C₁-C₈-Alkyl-, C₆-C₁₄-Aryl- und/oder C₂-C₈-Alkenylgruppen.

Die in der Definition der vorstehend genannten organischen Zinnverbindungen erwähnten linearen oder verzweigten, gegebenenfalls substituierten, C₁-C₃₀-Alkylgruppen schließen solche mit 1 bis 30 Kohlenstoffatomen ein, wie zum Beispiel Methyl, Ethyl, Chlorethyl, n-Propyl, isoPropyl, n-Butyl, iso-Butyl, Pentyl, Hexyl, Heptyl, Ethylhexyl, Octyl, Decyl, Undecyl, Dodecyl, Tridecyl, etc. Bevorzugt ist Butyl, Hexyl oder Octyl.

Die in der Definition der vorstehend genannten organischen Zinnverbindungen erwähnten C₅-C₁₄-Cycloalkylgruppen schließen mono-oder polycyclische Alkylgruppen ein, wie zum Beispiel Cyclopentyl, Cyclohexyl, Cyclohexylethyl Cyclooctyl, Decalinyl, Hydrindanyl, Bicyclo[2.2.1]heptanyl, Bicyclo[2.2.2]octanyl, Bicyclo[4.2.3]nonyl.

C₆-C₁₄-Arylgruppen umfassen z. B. Phenyl-, Naphthenyl- oder Fluorenylgruppen.

Bevorzugte Beispiele geeigneter Zinn-haltiger Verbindungen der Komponente III) sind Alkylzinnchloride und deren Mischungen, beispielsweise Di-n-butylzinndichlorid sowie Di-n-octylzinndichlorid, oder Alkylzinnoxide und deren Mischungen, beispielsweise Di-n-butylzinnoxid sowie Di-n-octylzinnoxid, Dibutylzinncarboxylate, beispielsweise Di-n-butylzinndiacetat, Di-n-butylzinndilaurat, Di-n-butylzinnmaleat, Di-n-butylzinn-bis-2-ethylhexanoat sowie Di-n-butylzinndineodecanoat, Dioctylzinncarboxylate, wie Di-n-octylzinndiacetat, Di-n-octylzinndilaurat, Di-n-octyl-zinnmaleat, Di-n-octylzinn-bis-2-ethylhexanoat oder Di-n-octylzinndineodecanoat, ferner Dialkylzinnkomplexe, beispielsweise Di-n-butylzinndiacetylacetonat. In den erfindungsgemäßen Mischungen sind in der Regel solche Zinnverbindungen besonders vorteilhaft einsetzbar, die sich in den erfindungsgemäßen Beschichtungsmitteln sofort oder nach Erwärmen lösen. Ganz besonders bevorzugte Verbindungen der Komponente III) sind Zinn-Ketonate. Die Menge der Zinn-haltigen Verbindung III) in dem erfindungsgemäßen Beschichtungsmittel beträgt bevorzugt 0,01 bis 1,0 Gew.-%, insbesondere bevorzugt 0,1 bis 1 Gew.-%, jeweils bezogen auf das Beschichtungsmittel.

Bei der Komponente IV) der erfindungsgemäßen Beschichtungsmittel handelt es sich um mindestens ein Aminosilan. Insbesondere handelt es sich bei dem mindestens einen Aminosilan um eines der allgemeinen Formel AₘSiYₙ, worin A eine substituierte oder unsubstituierte Aminoalkylgruppe, eine substituierte oder unsubstituierte Diaminodialkylgruppe oder eine substituierte oder unsubstituierte Triaminotrialkylgruppe darstellt, die Gruppen Y gleich oder verschieden sind, wobei Y für OH, ONa, OK, OR', OCOR', OSiR'₃, Cl, Br, I oder NR'₂ steht, m gleich 1 oder 2 und n gleich 1, 2 oder 3 sind, mit der Maßgabe m+n = 4, wobei die Gruppe R' unabhängig Wasserstoff, lineare oder verzweigte Alkyl-, Cycloalkyl-, Alkenyl-, Cycloalkenyl-, Alkinyl-, Cycloalkinyl-, Aryl- oder Heteroarylgruppen sind, jeweils 1 bis 18 C-Atomen aufweisen und jeweils ggf. substituiert sein können. Vorzugsweise ist m gleich 1 und n gleich 3. Weiterhin bevorzugt ist Y ausgewählt aus OH oder OR', wobei OR' besonders bevorzugt ist. In diesem Falle ist R' insbesondere ausgewählt aus Methyl- oder Ethylgruppen, wobei Methylgruppen insbesondere bevorzugt sind.

Bevorzugte Aminosilane sind die ausgewählt aus der Gruppe bestehend aus 3-Aminopropyltrimethoxysilan, 3-Aminopropyltriethoxysilan, 2- Aminoethyl-3-aminopropyltrimethoxysilan, 3-Aminopropyl(diethoxymethoxysilan), 3-Aminopropyl(tripropoxysilan), 3-Aminopropyl(dipropoxymethoxysilan), 3-Aminopropyl(tridodecanoxysilan), 3-Aminopropyl(tritetradecanoxysilan), 3-Aminopropyl(trihexadecanoxysilan), 3-Aminopropyl(trioctadecanoxysilan), 3-Aminopropyl(didodecanoxy)tetradecanoxysilan, 3-Aminopropyl(dodecanoxy)-tetradecanoxy(hexadecanoxy)silan, 3-Aminopropyl(dimethoxymethylsilan), 3-Aminopropyl(methoxydimethylsilan), 3-Aminopropyl(hydroxydimethylsilan), 3-Aminopropyl(diethoxymethylsilan), 3-Aminopropyl(ethoxydimethylsilan), 3-Aminopropyl(dipropoxymethylsilan), 3-Aminopropyl(propoxydimethylsilan), 3-Aminopropyl(diisopropoxymethylsilan), 3-Aminopropyl(isopropoxydimethylsilan), 3-Aminopropyl(dibutoxymethylsilan), 3-Aminopropyl(butoxydimethylsilan), 3-Aminopropyl(disiobutoxymethylsilan), 3-Aminopropyl(isobutoxydimethylsilan), 3-Aminopropyl(didodecanoxymethylsilan), 3-Aminopropyl(dodecanoxydimethylsilan), 3-Aminopropyl(ditetradecanoxymethylsilan), 3-Aminopropyl(tetradecanoxy-dimethylsilan), 2-Aminoethyl(trimethoxysilan), 2-Aminoethyl(triethoxysilan), 2-Aminoethyl(diethoxymethoxysilan), 2-Aminoethyl(tripropoxysilan), 2-Aminoethyl(dipropoxymethoxysilan), 2-Aminoethyl(tridodecanoxysilan), 2-Aminoethyl(tritetradecanoxysilan), 2-Aminoethyl(trihexadecanoxysilan), 2-Aminoethyl(trioctadecanoxysilan), 2-Aminoethyl(didodecanoxy)tetradecanoxysilan, 2-Aminoethyl(dodecanoxy)tetradecanoxy(hexadecanoxy)silan, 2-Aminoethyl(dimethoxymethylsilan), 2-Aminoethyl(methoxydimethylsilan), 2-Aminoethyl(diethoxymethylsilan), 2-Aminoethyl(ethoxydimethylsilan), 1-Aminomethyl(trimethoxysilan), 1-Aminomethyl(triethoxysilan), 1-Aminomethyl(diethoxymethoxysilan), 1-Aminomethyl(dipropoxymethoxysilan), 1-Aminomethyl(tripropoxysilan), 1-Aminomethyl(trimethoxysilan), 1-Aminomethyl(dimethoxymethylsilan), 1-Aminomethyl(methoxydimethylsilan), 1-Aminomethyl(diethoxymethylsilan), 1-Aminomethyl(ethoxydimethylsilan), 3-Aminobutyl(trimethoxysilan), 3-Aminobutyl(triethoxysilan), 3-Aminobutyl(diethoxymethoxysilan), 3-Aminobutyl(tripropoxysilan), 3-Aminobutyl(dipropoxymethoxysilan), 3-Aminobutyl(dimethoxymethylsilan), 3-Aminobutyl(diethoxymethylsilan), 3-Aminobutyl(dimethylmethoxysilan), 3-Aminobutyl(dimethylethoxysilan), 3-Aminobutyl(tridodecanoxysilan),3-Aminobutyl(tritetradecanoxysilan), 3-Aminobutyl(trihexadecanoxysilan), 3-Aminobutyl(didodecanoxy)tetradecanoxysilan, 3-Aminobutyl(dodecanoxy)tetra-decanoxy(hexadecanoxy)silan, 3-Amino-2-methyl-propyl(trimethoxysilan), 3-Amino-2-methyl-propyl(triethoxysilan), 3-Amino-2-methyl-propyl(diethoxymethoxysilan), 3-Amino-2-methyl-propyl(tripropoxysilan), 3-Amino-2-methyl propyl(dipropoxymethoxysilan), 3-Amino-2-methyl-propyl(tridodecanoxysilan), 3-Amino-2-methyl-propyl(tritetradecanoxysilan), 3-Amino-2-methyl-propyl(trihexadecanoxysilan), 3-Amino-2-methyl-propyl(trioctadecanoxysilan), 3-Amino-2-methyl-propyl(didodecanoxy)tetradecanoxysilan, 3-Amino-2-methyl-propyl(dodecanoxy)tetradecanoxy(hexadecanoxy)silan, 3-Amino-2-methyl-propyl(dimethoxymethylsilan), 3-Amino-2-methyl-propyl(methoxydimethylsilan), 3-Mercapto-2-methyl-propyl(diethoxymethylsilan), 3-Mercapto-2-methyl-propyl(ethoxydimethylsilan), 3-Mercapto-2-methyl-propyl(dipropoxymethylsilan), 3-Amino-2-methyl-propyl(propoxydimethylsilan), 3-Amino-2-methyl-propyl(diisopropoxymethylsilan), 3-Amino-2-methyl-propyl(isopropoxydimethylsilan), 3-Amino-2-methyl-propyl(dibutoxymethylsilan), 3-Amino-2-methyl-propyl(butoxydimethylsilan), 3-Amino-2-methyl-propyl(disiobutoxymethylsilan), 3-Amino-2-methyl-propyl(isobutoxydimethylsilan), 3-Amino-2-methyl-propyl(didodecanoxymethylsilan), 3-Amino-2-methyl-propyl(dodecanoxy-dimethylsilan), 3-Amino-2-methyl-propyl(ditetradecanoxymethylsilan) oder 3-Amino- 2-methyl-propyl (tetradecanoxydimethyl-silan), triaminofunktionelles Propyltri-methoxysilan, Bis(3-trimethoxysilylpropyl)-amin, Bis(3-triethoxysilylpropyl)-amin, N-Benzyl-N-(2-aminoethyl)-3-aminopropyltrimethoxysilan-Hydrochlorid, N-Benzyl-N-(2-aminoethyl)-3-aminopropyltrimethoxysilan-Hydroacetat, N-(n-Butyl)-3-aminopropyltrimethoxysilan, 3-Aminopropylmethyldiethoxysilan, N-Vinylbenzyl-N-(2- aminoethyl)-3-aminopropylpolysiloxan und N-(2-Aminoethyl)-3-aminopropylmethyldimethoxysilan. Bevorzugte Aminosilane oder Aminoalkylsilane sind substituierte oder unsubstituierte Aminosilan-Verbindungen, insbesondere 3-Aminopropyltrimethoxysilan, 3-Aminopropyltriethoxysilan, 3-Aminopropylmethyldiethoxysilan, N-(2-Aminoethyl)-3-aminopropyltrimethoxysilan, 2-Aminopropyl-3-aminopropyltrimethoxysilan, 2-Aminopropyl-3-aminopropyltriethoxysilan, 2-Aminoethyl-2-aminoethyl-3-aminopropyltrimethoxysilan, 2-Aminoethyl-2-aminoethyl-3-aminopropyltriethoxysilan und N-(n-Butyl)-3-aminopropyltrimethoxysilan.

Besonders bevorzugt ist das Aminosilan eines ausgewählt aus der Gruppe bestehend aus 3-Aminopropyltrimethoxysilan (DYNASYLAN® AMMO), 3-Aminopropyltriethoxysilan (DYNASYLAN® AMEO), 3-Aminopropylmethyldiethoxysilan (DYNASYLAN® 1505), N-(n-Butyl)-3-aminopropyltrimethoxysilan (DYNASYLAN® 1189) und N-(2-Aminoethyl)-3-aminopropyltrimethoxysilan (DYNASYLAN® DAMO), (H₃CO)₃Si(CH₂)₃NH(CH₂)₃Si(OCH₃)₃ (Bis-AMMO), (H₅C₂O)₃Si(CH₂)₃NH(CH₂)₃Si(OC₂H₅)₃ (Bis-AMEO), (H₃CO)₃Si(CH₂)₃NH(CH₂)₂NH(CH₂)₂NH(CH₂)₃Si(OCH₃)₃ (Bis-DAMO) (jeweils von Evonik Industries AG).

Die Menge an Komponente IV) in dem erfindungsgemäßen Beschichtungsmittel beträgt bevorzugt 5 bis 30 Gew.-%, insbesondere bevorzugt 10 bis 20 Gew.-%, jeweils bezogen auf das Beschichtungsmittel.

Die Herstellung der erfindungsgemäßen Beschichtungsmittel erfolgt durch Mischung der vorab beschriebenen Komponenten. Das Mischen kann in dem Fachmann bekannten Mischern erfolgen, beispielsweise Rührbehältern, Dissolvern, Perlmühlen, Walzenstühlen, etc., aber auch kontinuierlich mittels Statikmischern.

Gegenstand der vorliegenden Erfindung sind ebenfalls Metallbeschichtungszusammensetzungen, insbesondere für Automobilkarossen, Motor- und Fahrräder, Gebäudeteile und Haushaltsgeräte, welche die erfindungsgemäßen Copolymere oder Beschichtungsmittel enthalten.

Beschichtungszusammensetzungen für Glas-, Kunststoff-, Papier-, Textil- oder Holzbeschichtungen, insbesondere Klarlacke, enthaltend die erfindungsgemäßen Addukte oder Beschichtungsmittel sind ebenfalls Gegenstand der vorliegenden Erfindung. Die erfindungsgemäßen Beschichtungsmittel sind auch für die Mehrschichtlackierung, z. B. als Klarlack in der Automobil-Serienlackierung geeignet.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keinesfalls als in irgendeiner Weise limitierende Offenbarung aufzufassen. Nachfolgend wird die vorliegende Erfindung anhand von Beispielen näher erläutert. Alternative Ausführungsformen der vorliegenden Erfindung sind in analoger Weise erhältlich.

### Beispiele:

Sofern nicht anderes angegeben, beziehen sich die Mengenangaben in Prozent in den Beispielen auf das Gewicht.

### Beispiel a: Herstellung des erfindungsgemäßen Copolymers 1

In einem Dreihalskolben mit Rückflusskühler und Tropftrichter werden 21.09 g 1,5 Pentandiol, 0,01 g DBTL (Dibutylzinn(IV)-dilaurat) und 10g Butylacetat vorgelegt und unter Stickstoffatmosphäre auf 60°C erwärmt. Das zunächst trübe Gemisch wird bei ca. 55°C klar. Bei 60°C werden innerhalb von 1,5h 26,58 g H12MDI zugetropft, wobei das Reaktionsgemisch trübe ist. Nach Dosierende wird noch 30 min bei 60°C gerührt und dann der NCO-Gehalt bestimmt. Liegt der NCO-Gehalt bei <0,1%, so wird 42,23 g Isocyanatopropyltrimethoxysilan (IPMS) bei max. 60°C zugetropft. Nach 2,5h Zugabe ist die IPMS-Zugabe beendet und es werden noch 1 - 2h bei 60°C nachgerührt. Das resultierende Copolymer 1 ist eine bei Raumtemperatur trübe Flüssigkeit mit einer Viskosität (23°C) von 21 Pas (s.a. Tabelle 1).

### Beispiel b-e: Herstellung der erfindungsgemäßen Copolymere 2-5

Entsprechend Beispiel a werden die in den in Tabelle 1 angegebenen Mengen des Diols, des Katalysators und des Lösemittels Butylacetat vorgelegt und auf 60°C erwärmt. In die klare Lösung werden bei 60°C innerhalb von 1,5h die angegebene Menge Diisocyanat zugetropft. Nach 0,5 - 1h Reaktionszeit bei 60°C wird ein NCO-Gehalt von <0,1% NCO-Gehalt erreicht. Anschließend wird die angegebene Menge Isocyanatopropyltrimethoxysilan (IPMS) bei max. 60°C zugetropft. Nach 2,5h Zugabe ist die IPMS-Zugabe beendet und es werden noch 1 - 2h bei 60°C nachgerührt. Die Eigenschaften der resultierenden Copolymer 2-5 sind ebenfalls in Tabelle 1 angegeben.

**Tabelle 1: Übersicht zur Herstellung der erfindungsgemäßen Copolymere 1-5**

| **Beispiel** | **a** | **b** | **c** | **d** | **e** |
|---|---|---|---|---|---|
| **Copolymer** | **1** | **2** | **3** | **4** | **5** |
| Pentandiol | 21,5 % | 22,19 % | 22,39% | | |
| Hexandiol | | | | 24,4% | |
| Neopentylglykol | | | | | 22,08% |
| H12MDI | 26,58 % | | | | |
| IPDI | | 23,72 % | | 22,87% | 23,62 |
| TMDI | | | 22,66 % | | |
| Butylacetat | 10 | 10 | 10 | 10 | 10 |
| IPMS | 42,32 % | 44,07% | 44,93% | 42,89% | 44,30% |
| Reaktionszeit | 6h | 5h | 7h | 5h | 6h |

| **Eigenschaften** | | | | | |
|---|---|---|---|---|---|
| NCO-Gehalt | < 0,1 % | < 0,1 % | < 0,1 % | < 0,1 % | < 0,1 % |
| Viskosität (23°C) | 21 Pas | 14 Pas | 2,8 Pas | 12 Pas | 79 Pas |
| Aussehen | trüb | klar | trüb | klar | klar |

### Beispiel f: Herstellung der nicht-erfindungsgemäßen Copolymere 6-7

### f1a: Herstellung der Polyester-Vorstufen 6a

In einem Rührkessel mit Destillationsbrücke wurden 284g Pentandiol, 431 g Adipinsäure, 284 g Trimethylolpropan und 1 g Tegokat 256 vorgelegt. Unter Rühren wurde das Reaktionsgemisch aufgeheizt und bei 145 - 201 °C / 1013 - 165 mbar Druck wurde das bei der Veresterung entstehende Wasser abdestilliert. Nach 8,5h Reaktionszeit wurde die Polyester-Vorstufen 6a mit einer Hydroxylzahl von 348 mg KOH/g erhalten.

### f1b: Herstellung der Polyester-Vorstufen 7a

Es wurden 587 g Pentandiol, 412 g Adipinsäure und 1 g Tegokat 256 vorgelegt und die Veresterungsreaktion analog f1 a durchgeführt. Nach 6,5h Reaktionszeit wurde das Reaktionsgemisch mit einer Hydroxylzahl von 112 mg KOH/g erhalten. Dieses wurde mit 329,3 g Pentandiol versetzt. Nach Homogenisierung wurde die Polyester-Vorstufen 7a mit einer Hydroxylzahl von 373 mg KOH/g erhalten.

### f2a: Herstellung des nicht-erfindungsgemäßen Copolymers 6

Es wurde die 246,2 g Polyester-Vorstufen 6a und 0,17 g DBTL in einem Glaskolben mit Rückflusskühler und Tropftrichter vorgelegt und unter Rühren auf 50°C erhitzt. Anschließend wurden innerhalb von 3 h bei max. 70 °C 314,4 g Isocyanatopropyltrimethoxysilan (IPMS) zugetropft. Nach 3h Reaktionszeit bei 70°C wurde das Copolymer 6 mit einem NCO-Gehalt >0,1% erhalten.

### f2b: Herstellung des nicht-erfindungsgemäßen Copolymers 7

Es wurde die 243,6 g Polyester-Vorstufen 7a und 0,17 g DBTL in einem Glaskolben mit Rückflusskühler und Tropftrichter vorgelegt und unter Rühren auf 50°C erhitzt. Anschließend wurden innerhalb von 3 h bei max. 70 °C 333,2 g Isocyanatopropyltrimethoxysilan (IPMS) zugetropft. Nach 3h Reaktionszeit bei 70°C wurde das Copolmer 7 mit einem NCO-Gehalt >0,1% erhalten.

### Beispiel g: Herstellung des nicht-erfindungsgemäßen Copolymere 8

In einem Dreihalskolben mit Rückflusskühler und Tropftrichter werden 28,7 g Isocyanatopropyltrimethoxysilan (IPMS), 71,3 g OxyesterT1136 und 0,1% DBTL eingewogen mit Stickstoff überlagert und unter Rühren auf 60°C aufgeheizt. Nach ca. 15h Reaktionszeit bei 60°C wird ein NCO-Gehalt von <0,1% NCO-Gehalt erreicht. Das resultierende Copolymer 8 ist eine bei Raumtemperatur klare Flüssigkeit mit einer Viskosität (23°C) von 4 Pas (s.a. Tabelle 1).

### Beispiel h: Untersuchung der Materialeigenschaften verschiedener erfindungsgemäßer Klarlacke im Vergleich zu konventionellen 2K-PUR-Klarlacken und nicht-erfindungsgemäßen Klarlacken enthaltend Addukte aus Diolen bzw. Polyestern und Isocyanatopropyltrialkoxysilanen

Die erfindungsgemäßen Klarlacke (Zusammensetzungen: II, V, VI, VIII, IX, X) sowie der Vergleich auf Basis zweier 2K-PUR-Klarlacke (Zusammensetzungen: I, IV) und nicht-erfindungsgemäßen Klarlacken enthaltend Addukte aus Diolen bzw. Polyestern und Isocyanatopropyltrialkoxysilanen (Zusammensetzungen: III, VII, XI, XII) wurden gemäß den in der Tabelle 2 angegebenen Mengenteilen formuliert.

**Tabelle 2. Zusammensetzung der erfindungsgemäßen Klarlacken und Vergleichsbeispielen**

| Position | | I | II* | III | IV | V* | VI* | VII | VIII* | IX* | X* | XI | XII |
|---|---|---|---|---|---|---|---|---|---|---|---|---|---|
| 1 | Copolymer 1 | / | / | / | / | / | / | / | / | / | 89,9 | / | / |
| 2 | Copolymer 2 | / | 39,58 | / | / | / | / | / | 26,73 | / | / | / | / |
| 3 | Copolymer 3 | / | / | / | / | / | / | / | / | 28,02 | / | / | / |
| 4 | Copolymer 4 | / | / | / | / | / | 24,78 | / | / | / | / | / | / |
| 5 | Copolymer 5 | / | / | / | / | 24,78 | / | / | / | / | / | / | / |
| 6 | Copolymer 6 | / | / | / | / | / | / | 25,38 | / | / | / | / | / |
| 7 | Copolymer 7 | / | / | 26,93 | / | / | / | / | / | / | / | / | / |
| 8 | Copolymer 8 | / | / | / | / | / | / | / | / | / | / | 25,0 | / |
| 9 | Dynasilan AMMO | / | / | / | / | / | / | / | / | / | 10 | 10 | 10 |
| 10 | VESTANAT EP-M 95 | / | / | / | / | / | / | / | / | / | / | 64,9 | 89,9 |
| 11 | Setalux^{®} 1767 W-65 (65 %ig) | 52,20 | / | / | / | / | / | / | / | / | / | / | / |
| 12 | Setalux^{®} 1760 VB-64 (64 %ig) | / | 23,86 | 42,08 | 49,47 | 34,85 | 34,85 | 39,65 | 37,60 | 39,40 | / | / | / |
| 13 | VESTANAT^{®} HT 2500 L (90 %ig) | 19,24 | / | / | 14,51 | / | / | / | / | / | / | / | / |
| 14 | TEAB | / | 0,51 | 0,54 | / | 0,45 | 0,45 | 0,76 | 0,96 | 1,01 | / | / | / |
| 15 | DBTL | / | / | / | / | / | / | / | / | / | 0,1 | 0,1 | 0,1 |
| 16 | Byketol^{®} spezial | 2,60 | / | / | / | / | / | / | / | / | / | / | / |
| 17 | Byk^{®} 301 | 0,20 | / | / | / | / | / | / | / | / | / | / | / |
| 18 | Tego^{®} Glide 410 | / | 0,05 | 0,05 | 0,05 | 0,05 | 0,05 | 0,05 | 0,05 | 0,05 | / | / | / |
| 19 | Tinuvin^{®} 292 | 0,26 | 0,25 | 0,27 | 0,22 | 0,22 | 0,22 | 0,25 | 0,24 | 0,24 | / | / | / |
| 20 | Tinuvin^{®} 900 | 3,26 | 3,19 | 3,37 | 2,80 | 2,76 | 2,76 | 3,17 | 2,98 | 3,04 | / | / | / |
| 21 | Butylacetat / Xylol-Gemisch (1:1) | 22,24 | 32,56 | 26,76 | 32,95 | 36,89 | 36,89 | 30,74 | 31,44 | 28,24 | | | |

| | | | | | | | | | | | | | |
|---|---|---|---|---|---|---|---|---|---|---|---|---|---|
| *) erfindungsgemäß **Setalux® 1767 VVI-65:** Polyacrylatpolyol, Nuplex Resins B.V. **Setalux® 1760 VB-64:** Polyacrylatpolyol, Nuplex Resins B.V. **Byk® 301:** Polyethermodifiziertes Polydimethylpolysiloxan, Verlaufsmittel, Byk Chemie **Byketol® Special:** Verlaufsmittel auf Basis hochsiedender Lösemittel sowie polyethermodifizierter Polydimethylsiloxane, Byk Chemie **Tinuvin® 292:** sterisch gehindertes Amin, Lichtschutzmittel; BASF SE **Tinuvin® 900:** UV-Absorber; BASF SE | | | | | | | | | | | | | |

Sämtliche 2K PUR Klarlacke (Zusammensetzungen I, IV) wurden als 2-Komponenten-Systeme formuliert, d. h. die Härterkomponente (Position 13) und die Polyolkomponenenten (Position 11-12) wurden unmittelbar vor der Verarbeitung gemischt. Im Fall der erfindungsgemäßen Klarlacke (Zusammensetzungen II, V, VI, VIII, IX) wurde gleichartig vorgegangen. Die erfindungsgemäßen Copolymere 2-5 wurden mit der Polyolkomponente (Position 12) unmittelbar vor Verarbeitung gemischt. Im Fall der erfindungsgemäßen Klarlacke (Zusammensetzungen X) wurde das erfindungsgemäße Copolymer 1 mit einem Aminosilan (Position 9) einkomponentig kombiniert. Eine gleichartige, einkomponentige Kombination mit einem Addukt, aus Diolen und IPMS (Position 10) und einem Aminosilan (Position 9) wurde bei den Zusammensetzungen XI und XII realisiert.

Die Viskosität der Formulierungen betrug, bestimmt als Auslaufzeit im DIN-4-Becher bei 23 °C, ca. 20 Sekunden.

Zur Ermittlung der mechanischen Kenndaten wurden alle Lacke im druckluftunterstützten Spritzauftrag mittels einer HPLV-Pistole auf phosphatierte Stahlbleche (Chemetall Gardobond 26S/60/OC) appliziert und bei unterschiedlichen Einbrennbedingungen (Raumtemperatur, 30' 80°C,22'140°C) gehärtet.

Die **Prüfung auf Kratzbeständigkeit** erfolgte im Zweischichtaufbau, des Klarlacks über einem schwarzen Basislack. Dazu wurde ein wässriger schwarzer Basislack (Autowave MM 245, tiefschwarz; 100:5 mit Aktivator WB abgemischt, Härtung: 15' 50°C) im Spritzauftrag bei Säurebeständigkeit auf Prüfbleche aus Sondertiefziehmaterial mit RP-Oberfläche nach DIN 1624, 570 x 98 x 0,8 mm und bei Kratzbeständigkeit auf Karosseriebleche (Stahl, 190 x 105 x 0,8 mm DIN 1624) appliziert und nach einer Ablüftzeit von 10 Min. bei Raumtemperatur im Umluftofen für 10 Min. bei 80°C getrocknet. Die Trockenfilmschichtdicke beträgt jeweils ca. 10 µm.

Zur **Prüfung auf Kratzbeständigkeit** wurde ein 45 mm x 20 mm Nylongewebe, Maschenweite 25 µm, wird mit einem 2 Kg-Gewicht beschwert, auf die Prüfplatte, die ihrerseits auf einem Schlitten fixiert ist, aufgelegt und arretiert. Nach Auftrag von 1 ml einer aufgerührten, 0,25%-igen Waschmittellösung (Persil) unmittelbar vor der Prüffläche wird die Prüfplatte mit einer maximalen Auslenkung von jeweils ca. 3,5 cm oszilliert. Nach 80 Doppelhüben (1 s⁻¹) wird die verbliebene Waschflüssigkeit mit Leitungswasser abgespült und mit Druckluft getrocknet.

Jeweils vor und nach der Prüfung werden Glanzmessungen (20°-Winkel) vorgenommen.

**Tabelle 3: Eigenschaften der Klarlacke I bis III**

| | Zusammensetzung | | |
|---|---|---|---|
| | I | II* | III |
| Härtung | 22' 140°C | 22'140°C | 22'140°C |
| Pendelhärte (König) [s] n7d | 171 | 184 | 70 |
| Kugelschlag [In Ibs] (DIN-EN-ISO 6272-1 | 80 | > 80 | > 80 |
| MEK-Test [ASTM D 4752] (Doppelhübe, 1 kg Auflagegewicht) | > 150 | >150 | > 150 |
| Kratzbeständigkeit Ausgangsglanz / Glanzverlust [Skt] | 79 / 20 | 83/7 | 84/0 |

| | | | |
|---|---|---|---|
| *) erfindungsgemäß | | | |

Die Ergebnisse in Tabelle 3 belegen, dass der erfindungsgemäße Klarlack (Zusammensetzung II), eine hervorragende Kratzbeständigkeit aufweist und dem 2K-PUR-Lack (Zusammensetzung I) diesbezüglich weit überlegen ist. Zusammensetzung III enthaltend ein Addukt aus einem Polyester und Isocyanatopropyltrialkoxysilan (Copolymer 7) ist im Vergleich zum erfindungsgemäßen Beschichtungsmittel wesentlich weicher und in diesem Punkt deutlich unterlegen.

**Tabelle 4: Eigenschaften der Klarlacke IV bis VII**

| | Zusammensetzung | | | |
|---|---|---|---|---|
| | IV | V* | VI* | VII |
| Härtung | 30'80°C | 30' 80°C | 30' 80°C | 30'80°C |
| Pendelhärte (König) [s] n7d | 188 | 195 | 186 | 72 |
| Kugelschlag [In Ibs] (DIN-EN-ISO 6272-1 | > 80 | 60 | 80 | 80 |
| MEK-Test [ASTM D 4752] (Doppelhübe, 1kg Auflagegewicht) | > 150 | > 150 | > 150 | >150 |
| Kratzbeständigkeit Ausgangsglanz / Glanzverlust [Skt] | 87 /44 | 87/21 | 88/14 | / |

| | | | | |
|---|---|---|---|---|
| *) erfindungsgemäß | | | | |

Die Ergebnisse in Tabelle 4 belegen, dass die erfindungsgemäßen Klarlacke (Zusammensetzung V, VI), eine hervorragende Kratzbeständigkeit aufweisen und dem 2K-PUR-Lack (Zusammensetzung IV) diesbezüglich weit überlegen sind. Zusammensetzung VII enthaltend ein Addukt aus einem Polyester und Isocyanatopropyltrialkoxysilan (Copolymer 6) ist im Vergleich zu den erfindungsgemäßen Klarlacken wesentlich weicher und in diesem Punkt deutlich unterlegen.

**Tabelle 5: Eigenschaften der Klarlacke IV, VIII und IX**

| | Zusammensetzung | | |
|---|---|---|---|
| | IV | VIII* | IX* |
| Härtung | RT | RT | RT |
| Pendelhärte (König) [s] n7d | 127 | 120 | 154 |
| Kugelschlag[ln Ibs] (DIN-EN-ISO 6272-1 | > 80 | 80 | 50 |
| | | | |
| MEK-Test [ASTM D 4752] (Doppelhübe, 1 kg Auflagegewicht) | 135 | >150 | > 150 |
| Kratzbeständigkeit Ausgangsglanz / Glanzverlust [Skt] | 90/ 40 | 85/17 | 90/32 |

| | | | |
|---|---|---|---|
| *) erfindungsgemäß | | | |

Die Ergebnisse in Tabelle 5 belegen, dass die erfindungsgemäßen Klarlacke (Zusammensetzung VIII, IX), eine dem 2K-PUR-Lack (Zusammensetzung IV) überlegene Kratzbeständigkeit aufweisen und in den Punkten Härte, Flexibilität sowie Chemikalienbeständigkeit auf gleichem Niveau liegen.

**Tabelle 6: Eigenschaften der Klarlacke X bis XII**

| | Zusammensetzung | | |
|---|---|---|---|
| | X* | XI | XII |
| Härtung | RT | RT | RT |
| Pendelhärte (König) [s] n7d | 120 | 108 | 134 |
| Erichsen Tiefung [mm] (EN ISO 1520) | 5 | 2,5 | 1,5 |
| MEK-Test [ASTM D 4752] (Doppelhübe, 1 kg Auflagegewicht) | >150 | >150 | > 150 |

| | | | |
|---|---|---|---|
| *) erfindungsgemäß | | | |

Die Ergebnisse in Tabelle 6 belegen, dass der erfindungsgemäße Klarlack (Zusammensetzung X), welcher wie Zusammensetzung XI und XII ein einkomponentiges System darstellt, im Vergleich mit diesen Zusammensetzungen das ausgewogenste Eigenschaftsbild zeigt. Auch hier kann neben einer vergleichbar hohen Härte gleichzeitig eine hohe Flexibilität der Beschichtung erzielt werden, was unter Nichtverwendung des erfindungsgemäßen Copolymer 1 verwehrt wird.

## Patentansprüche

1. Copolymere der Formel 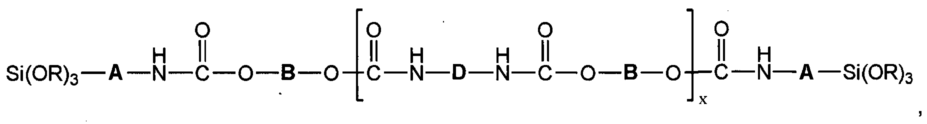 mit
A, B, D unabhängig voneinander = aliphatischer (Cyclo)Alkylenrest,
R = C₁-C₁₀-Alkylrest, und
3 ≤ x ≤ 10.

2. Copolymer nach Anspruch 1, **dadurch gekennzeichnet, dass** jedes A ein linearer C₁-C₁₀-Alkylenrest ist.

3. Copolymer nach Anspruch 1 oder 2, **dadurch gekennzeichnet, dass** jedes R ein Methyl-, Ethyl- oder i-Propylrest ist.

4. Copolymer nach einem der vorhergehenden Ansprüche, **dadurch gekennzeichnet, dass** jedes B ein linearer, verzweigter oder cyclischer C₁-C₁₂-Alkylenrest ist.

5. Copolymer nach einem der vorhergehenden Ansprüche, **dadurch gekennzeichnet, dass** jedes D ein (cyclo)aliphatischer C₆-C₁₅Alkylenrest ist.

6. Copolymer nach Anspruch 5, **dadurch gekennzeichnet, dass** jedes D eine Strukturformel ausgewählt aus der Gruppe an Formeln bestehend aus 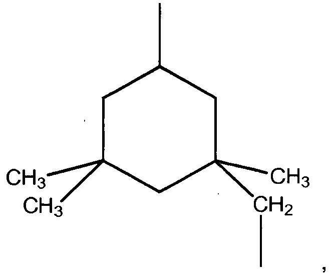 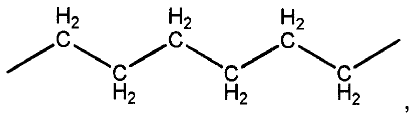 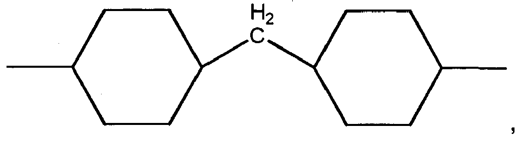 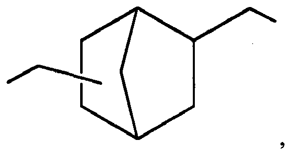 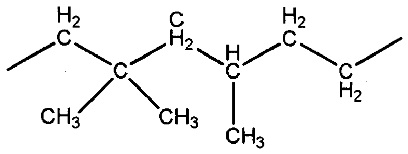 und 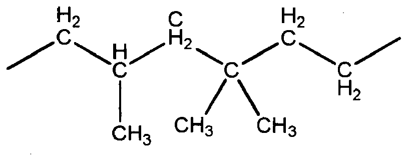 aufweist.

7. Verfahren zur Herstellung eines Copolymers nach einem der vorhergehenden Ansprüche, bei dem mindestens ein Diol mit mindestens einem Diisocyanat zu einem Urethanzwischenprodukt umgesetzt wird, welches anschließend mit mindestens einem Isocyanatoalkyltrialkoxysilan umgesetzt wird.

8. Verwendung eines Copolymers nach einem der Ansprüche 1 - 6 als Beschichtungsmittel oder als Bestandteil von Beschichtungsmitteln.

9. Beschichtungsmittel, enthaltend
A) mindestens ein Copolymer nach einem der Ansprüche 1 - 6,
B) eine oder mehrere Bindemittelkomponenten,
C) optional bis zu 4 Gew.-% mindestens eines Katalysators,
D) optional Hilfs- und Zusatzstoffe,
E) optional organische Lösemittel.

10. Beschichtungsmittel gemäß Anspruch 9, **dadurch gekennzeichnet, dass** die Komponente B) ausgewählt wird aus der Gruppe bestehend aus Hydroxylgruppen-haltigen Polyestern, Polyethern, Poly(meth)acrylaten, Polycarbonaten und Polyurethanen mit einer OH-Zahl von 20 bis 500 mg KOH/g und einer mittleren Molmasse von 250 bis 6000 g/Mol.

11. Beschichtungsmittel gemäß einem der Ansprüche 9 und 10, **dadurch gekennzeichnet, dass** die Komponente C) mindestens ein Katalysator ausgewählt aus der Gruppe C1) der organischen Carbonsäuren mit einem Schmelzpunkt oberhalb von 60 °C und/oder der Gruppe C2) der Tetraalkylammoniumcarboxylate ist.

12. Beschichtungsmittel gemäß Anspruch 11, **dadurch gekennzeichnet, dass** die Komponente C2) ausgewählt ist aus Tetramethylammoniumformiat, Tetramethylammoniumacetat, Tetramethylammoniumpropionat, Tetramethylammoniumbutyrat, Tetramethylammoniumbenzoat, Tetraethylammoniumformiat, Tetraethylammoniumacetat, Tetraethylammoniumpropionat, Tetraethylammoniumbutyrat, Tetraethylammoniumbenzoat, Tetrapropylammoniumformiat, Tetrapropylammoniumacetat, Tetrapropylammoniumpropionat, Tetrapropylammoniumbutyrat, Tetrapropylammoniumbenzoat, Tetrabutylammoniumformiat, Tetrabutylammoniumacetat, Tetrabutylammoniumpropionat, Tetrabutylammoniumbutyrat und/oder Tetrabutylammoniumbenzoat.

13. Beschichtungsmittel enthaltend
I) mindestens ein Copolymer nach einem der Ansprüche 1-6
II) optional mindestens ein Addukt aus mindestens einem Isocyanatosilan und mindestens einer Hydroxy-funktionellen Verbindung,
III) mindestens eine Zinn-haltige Verbindung und
IV) mindestens ein Aminosilan.

14. Metallbeschichtungszusammensetzungen und Beschichtungszusammensetzungen für Glas-, Kunststoff-, Papier- Textil- oder Holzbeschichtungen enthaltend mindestens ein Copolymer nach einem der Ansprüche 1 - 6 oder mindestens ein Beschichtungsmittel nach einem der Ansprüche 9 bis 13.

## Claims

1. Copolymers of the formula 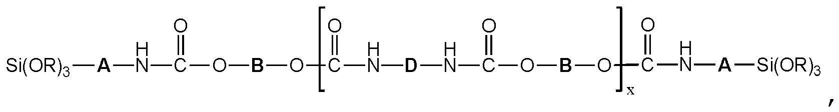 where
A, B, D independently of one another = aliphatic (cyclo)alkylene radical,
R = C₁-C₁₀ alkyl radical, and
3 ≤ x ≤ 10.

2. Copolymer according to Claim 1, **characterized in that** each A is a linear C₁-C₁₀ alkylene radical.

3. Copolymer according to Claim 1 or 2, **characterized in that** each R is a methyl, ethyl or isopropyl radical.

4. Copolymer according to any of the preceding claims, **characterized in that** each B is a linear, branched or cyclic C₁-C₁₂ alkylene radical.

5. Copolymer according to any of the preceding claims, **characterized in that** each D is a (cyclo)aliphatic C₆-C₁₅ alkylene radical.

6. Copolymer according to Claim 5, **characterized in that** each D has a structural formula selected from the group of formulae consisting of 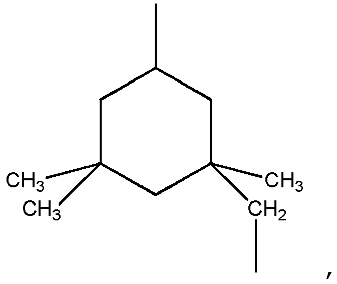 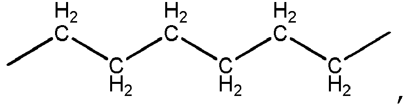 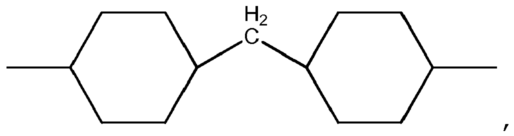 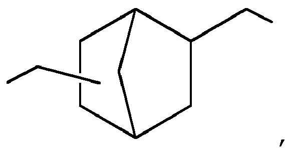 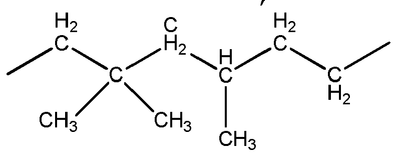 and

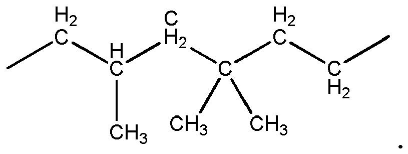

7. Process for preparing a copolymer according to any of the preceding claims, wherein at least one diol is reacted with at least one diisocyanate to give a urethane intermediate, which is subsequently reacted with at least one isocyanatoalkyltrialkoxysilane.

8. Use of a copolymer according to any of Claims 1 - 6 as coating composition or as constituent of coating compositions.

9. Coating composition comprising
A) at least one copolymer according to any of Claims 1 - 6,
B) one or more binder components,
C) optionally up to 4 wt% of at least one catalyst,
D) optionally auxiliaries and additives,
E) optionally organic solvents.

10. Coating composition according to Claim 9, **characterized in that** component B) is selected from the group consisting of hydroxyl-containing polyesters, polyethers, poly(meth)acrylates, polycarbonates and polyurethanes having an OH number of 20 to 500 mg KOH/g and an average molar mass of 250 to 6000 g/mol.

11. Coating composition according to either of Claims 9 and 10, **characterized in that** component C) is at least one catalyst selected from the group C1) of the organic carboxylic acids having a melting point above 60°C and/or the group C2) of the tetraalkylammonium carboxylates.

12. Coating composition according to Claim 11, **characterized in that** component C2) is selected from tetramethylammonium formate, tetramethylammonium acetate, tetramethylammonium propionate, tetramethylammonium butyrate, tetramethylammonium benzoate, tetraethylammonium formate, tetraethylammonium acetate, tetraethylammonium propionate, tetraethylammonium butyrate, tetraethylammonium benzoate, tetrapropylammonium formate, tetrapropylammonium acetate, tetrapropylammonium propionate, tetrapropylammonium butyrate, tetrapropylammonium benzoate, tetrabutylammonium formate, tetrabutylammonium acetate, tetrabutylammonium propionate, tetrabutylammonium butyrate and/or tetrabutylammonium benzoate.

13. Coating composition comprising
I) at least one copolymer according to any of Claims 1 - 6,
II) optionally at least one adduct of at least one Isocyanatosilane and at least one hydroxy-functional compound,
III) at least one tin-containing compound and
IV) at least one aminosilane.

14. Metal-coating formulations and coating formulations for glass, plastic, paper, textile or wood coatings, comprising at least one copolymer according to any of Claims 1 - 6 or at least one coating composition according to any of Claims 9 to 13.

## Revendications

1. Copolymères de formule 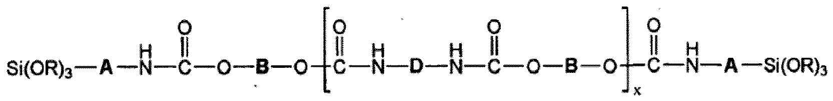 avec
A, B, D, indépendamment les uns des autres = radical (cyclo)alkylène aliphatique,
R = radical alkyle en C₁-C₁₀ et
3 ≤ x ≤ 10.

2. Copolymère selon la revendication 1, **caractérisé en ce que** chaque A est un radical alkylène en C₁-C₁₀ linéaire.

3. Copolymère selon la revendication 1 ou 2, **caractérisé en ce que** chaque R est un radical méthyle, éthyle ou i-propyle.

4. Copolymère selon l'une quelconque des revendications précédentes, **caractérisé en ce que** chaque B est un radical alkylène en C₁-C₁₂ linéaire, ramifié ou cyclique.

5. Copolymère selon l'une quelconque des revendications précédentes, **caractérisé en ce que** chaque D est un radical alkylène en C₆-C₁₅ (cyclo)aliphatique.

6. Copolymère selon la revendication 5, **caractérisé en ce que** chaque D présente une formule structurale choisie dans le groupe de formules constitué par 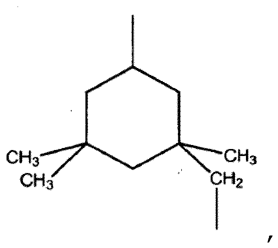 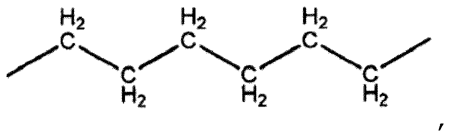 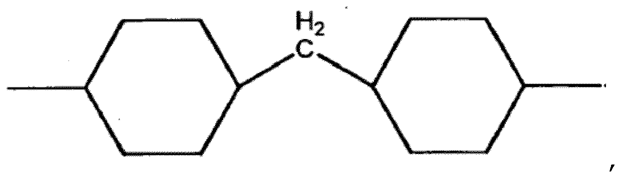 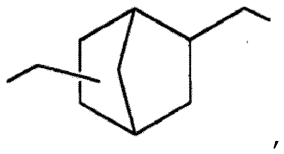 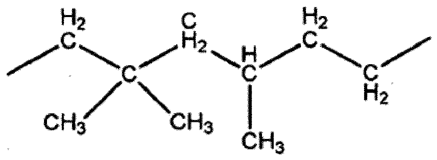 et

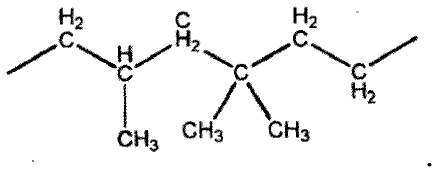

7. Procédé de fabrication d'un copolymère selon l'une quelconque des revendications précédentes, selon lequel au moins un diol est mis en réaction avec au moins un diisocyanate pour former un produit intermédiaire uréthane, qui est ensuite mis en réaction avec au moins un isocyanatoalkyltrialcoxysilane.

8. Utilisation d'un copolymère selon l'une quelconque des revendications 1 à 6 en tant qu'agent de revêtement ou en tant que constituant d'agents de revêtement.

9. Agent de revêtement, contenant :
A) au moins un copolymère selon l'une quelconque des revendications 1 à 6,
B) un ou plusieurs composants liants,
C) éventuellement jusqu'à 4 % en poids d'au moins un catalyseur,
D) éventuellement des adjuvants et additifs,
E) éventuellement des solvants organiques.

10. Agent de revêtement selon la revendication 9, **caractérisé en ce que** le composant B) est choisi dans le groupe constitué par les polyesters contenant des groupes hydroxyle, les polyéthers, les poly(méth)acrylates, les polycarbonates et les polyuréthanes ayant un indice OH de 20 à 500 mg KOH/g et une masse molaire moyenne de 250 à 6 000 g/mol.

11. Agent de revêtement selon l'une quelconque des revendications 9 et 10, **caractérisé en ce que** le composant C) est au moins un catalyseur choisi dans le groupe C1) constitué par les acides carboxyliques organiques ayant un point de fusion supérieur à 60 °C et/ou le groupe C2) constitué par les carboxylates de tétraalkylammonium.

12. Agent de revêtement selon la revendication 11, **caractérisé en ce que** le composant C2) est choisi parmi le formiate de tétraméthylammonium, l'acétate tétraméthylammonium, le propionate de tétraméthylammonium, le butyrate de tétraméthylammonium, le benzoate de tétraméthylammonium, le formiate de tétraéthylammonium, l'acétate de tétraéthylammonium, le propionate de tétraéthylammonium, le butyrate de tétraéthylammonium, le benzoate de tétraéthylammonium, le formiate de tétrapropylammonium, l'acétate de tétrapropylammonium, le propionate de tétrapropylammonium, le butyrate de tétrapropylammonium, le benzoate de tétrapropylammonium, le formiate de tétrabutylammonium, l'acétate de tétrabutylammonium, le propionate de tétrabutylammonium, le butyrate de tétrabutylammonium et/ou le benzoate de tétrabutylammonium.

13. Agent de revêtement, contenant :
I) au moins un copolymère selon l'une quelconque des revendications 1 à 6,
II) éventuellement au moins un adduit d'au moins un isocyanatosilane et d'au moins un composé à fonction hydroxy,
III) au moins un composé contenant de l'étain, et
IV) au moins un aminosilane.

14. Compositions de revêtement métallique ou compositions de revêtement pour revêtements en verre, plastique, papier, textile ou bois, contenant au moins un copolymère selon l'une quelconque des revendications 1 à 6 ou au moins un agent de revêtement selon l'une quelconque des revendications 9 à 13.
